# EUROPÄISCHE PATENTANMELDUNG

(11) **EP 1 886 641 A1**
(43) Veröffentlichungstag der Anmeldung: **13.02.2008**
(21) Anmeldenummer: 06016809.3
(22) Anmeldetag: 11.08.2006
(51) Int. Cl.: A61B 19/00, A61B 17/16

(54) **Verfahren und System zum Bestimmen der relativen Lage eines medizinischen Instruments relativ zu einer Körperstruktur**

(71) Anmelder: BrainLAB AG, 85622 Feldkirchen (DE)
(72) Erfinder: Tuma, Gregor, 81675 München (DE)
(74) Vertreter: Schwabe - Sandmair - Marx

(57) **Zusammenfassung**

Verfahren zum Bestimmen der relativen Lage eines Gegenstandes (2) relativ zu einer Körperstruktur, insbesondere zum Bestimmen der relativen Lage eines medizinischen Instruments relativ zu einer Körperstruktur (5), insbesondere auch wenn sich das Instrument teilweise innerhalb der Körperstruktur (5) befindet; wobei das Verfahren die folgenden Schritte umfasst:
a) die relative Lage (C) eines ersten Teils (P1,P2,P3) des Gegenstandes (2) zu einem zweiten, vom ersten Teil verschiedenen Teil (P4,2c) des Gegenstandes (2) wird bereitgestellt,
b) eine Markereinrichtung (4) wird an der Körperstruktur (5) angebracht;
c) die relative Lage (A) der Markereinrichtung (4) relativ zur Körperstruktur (5) wird erfasst;
d) der Gegenstandes (2) wird relativ zur Körperstruktur (5) fixiert;
e) die relative Lage (B) des ersten Teils (P1,P2,P3) des Gegenstandes (2) relativ zur Markereinrichtung (4) wird erfasst;
f) die relative Lage (D) des zweiten Teils (P4,2c) des Gegenstandes (2) relativ zur Lage der Körperstruktur (5) wird basierend auf der relativen Lage (C) des ersten Teils (P1,P2,P3) relativ zum zweiten Teil (P4,2c), basierend auf der relativen Lage (A) der Markereinrichtung (4) relativ zur Körperstruktur (5) und basierend auf der relativen Lage (B) des ersten Teils (P1,P2,P3) relativ zur Markeranordnung (4) berechnet.
Ein System zur Ausführung des Verfahrens ist ebenfalls offenbart.

## Beschreibung

Die vorliegende Erfindung betrifft ein Verfahren und ein System zum Bestimmen der relativen Lage eines medizinischen Instruments relativ zu einer Körperstruktur. Insbesondere dient die Bestimmung der relativen Lage dem Positionieren eines Gegenstandes, insbesondere eines medizinischen Instruments, das teilweise in einer Körperstruktur, insbesondere in einem Knochen ist. Im medizinischen Bereich, insbesondere bei Operationen, können Situationen gegeben sein, bei denen eine Information über die relative Lage eines Teils eines Instruments relativ zu einer Körperstruktur nicht direkt oder nur schwierig direkt erfassbar ist. Wird beispielsweise ein Instrument in eine Körperstruktur eingebracht und verbieten die engen Platzverhältnisse das Anbringen eines Markers oder eines Referenzsterns an dem Instrument, so stellt es ein Problem dar, die Position von verdeckten Teilen des Instruments relativ zu der Körperstruktur zu bestimmen.

Aufgabe der Erfindung ist es die Lage eines Gegenstandes relativ zu einer Körperstruktur zu bestimmen.

Vorstehende Aufgabe wird durch die Gegenstände der unabhängigen Ansprüche gelöst. Die abhängigen Ansprüche sind auf vorteilhafte Ausführungsformen gerichtet.

Das erfindungsgemäße Verfahren umfasst vorzugsweise mehrere Schritte, die vorzugsweise aber nicht zwingend in der im Folgenden beschriebenen Reihenfolge durchgeführt werden.

Der hierin verwendete Begriff Körperstruktur umfasst insbesondere Knochen und Knorpel oder auch künstliche Gliedmaßen oder Implantate. Der hierin verwendete Begriff "Instrument" umfasst sowohl eine einteilige (d.h. das Instrument besteht aus einem einzigen Element) als auch eine mehrteilige (d.h. das Instrument besteht aus zwei oder mehr Elementen) Ausführung eines Instruments. Das Instrument ist ein Beispiel für einen (körperfremden) Gegenstand. Bei dem Gegenstand kann es sich beispielsweise um ein medizinisches Instrument, insbesondere auch ein resorbierbares Implantat, oder eine (andere) Körperstruktur handeln. Der im Folgenden verwendete Begriff "Instrument" steht beispielhaft für den vorgenannten (insbesondere körperfremden) Gegenstand.

Der Begriff "Lagemerkmal" bezeichnet im folgenden eine vorbestimmte, insbesondere charakteristische, relative Lage eines Teils eines Objekts (z.B. Gegenstand oder Körperstruktur) relativ zur Lage eines anderen Teils desselben Objekts oder relativ zur Lage eines Teils eines anderen Objekts. Die Lage eines Teils wird bevorzugt durch eine oder mehrere Positionen des Teils in einem vorbestimmten Bezugssystem bestimmt. Zur Beschreibung der Lage eines punktförmigen Teils (z.B. einer Spitze eines Instruments) genügt vorzugsweise eine Position. Zur Beschreibung der Lage einer Achse werden vorzugsweise mindestens zwei Positionen verwendet. Zur Beschreibung der Lage einer Ebene werden vorzugsweise mindestens drei Positionen verwendet. Bei dem vorgenannten Begriff "Teil" kann es sich z.B. um Oberflächenabschnitte (z.B. punktförmig oder in der Gestalt einer ebenen oder gekrümmten Fläche), um Ebenen oder um Achsen (z.B. die Längsachse des Instruments oder eines Knochens) handeln. Die Positionen können z.B. mit kartesischen Koordinaten oder Kugelkoordinaten bestimmt werden. Die relative Lage von einem Teil relativ zu einem anderen Teil kann insbesondere durch Raumwinkel und/oder Abstände und/oder Koordinaten (in einem Bezugssystem) und/oder Vektoren beschrieben werden und wird vorzugsweise aus den die Lage beschreibenden Positionen z.B. mittels eines Programms errechnet, das auf einem Computer läuft.

Der hierin verwendete Begriff "relative Lage" oder der Ausdruck "Lage eines ersten Teils A relativ zu einem zweiten Teil B" umfasst also den Begriff der relativen Position zwischen den zwei Teilen, insbesondere punktförmigen Teilen. Ist die Position eines Teils in einem Bezugssystem bekannt, so kann basierend auf der relativen Lage zweier Teile aus der Position eines der zwei Teile die Position des anderen der zwei Teile berechnet werden. Dies gilt insbesondere für punktförmige Teile. Weiter ist natürlich auch der Fall der relativen Lage beispielsweise zwischen zwei als Ebenen ausgebildeten Teilen umfasst. Die relative Lage bezeichnet hier beispielsweise den Winkel, den die Ebenen zueinander einnehmen. Für bestimmte Zwecke ist ein derartiger Winkel ausreichend und eine Abstandsbestimmung der Ebenen nicht unbedingt notwendig. Der Begriff "relative Lage" muss natürlich in dem Fall von Ebenen nicht auf den relativen Winkel der Ebenen zueinander beschränkt sein (insbesondere Raumwinkel) sondern kann auch, z.B. durch Festlegung eines Mittelpunktes der Ebenen, einen Abstand zwischen den Mittelpunkten der Ebenen umfassen. Der Begriff "relative Lage" kann insbesondere eine Information über die relative Position zweier Teile zueinander und/oder deren Orientierung und/oder deren Abstand enthalten.

Bevorzugt ist mindestens ein Lagemerkmal des Gegenstandes bekannt und messbar. Das oder die Lagemerkmale sind insbesondere, z.B. in einem Datenverarbeitungssystem (Computer) gespeichert.

Sollten das oder die Lagemerkmale des Gegenstandes bzw. die relative Lage zwischen dem ersten und zweiten Teil nicht bekannt sein, insbesondere nicht gespeichert sein, so kann es bzw. können sie mittels einer Messeinrichtung für die weitere Verarbeitung (insbesondere Datenverarbeitung) bestimmt und insbesondere auch gespeichert werden. Die Messeinrichtung kann optische Messverfahren ersetzen, bei denen z.B. mittels Pointer die Positionen einzelner Teile des Gegenstands (in einem bestimmten Bezugssystem) abgetastet bzw. erfasst werden. Dies kann optisch mittels einer Kamera erfolgen, wobei passiv reflektierende Marker oder aktiv emmitierende Marker, die z.B. am Pointer angebracht sind. verwendet werden. Auch kann eine Bestimmung der Lage von Teilen, also beispielsweise von Oberflächenabschnitten, von Punkten auf der Oberfläche oder von Ebenen relativ zu anderen Teilen nicht nur durch die vorgenannten Pointer und/oder optische Messverfahren erfolgen sondern beispielsweise auch durch Ultraschallmessungen oder NMR (Kernspinresonanz) -Messungen oder Röntgen-CT-Messung, die eine dreidimensionale Rekonstruktion des zu vermessenden Gegenstandes erlauben. Ebenfalls kann der Gegenstand dreidimensional optisch durch mehrere Kameras oder verfahrbare Kameras, insbesondere auf einem vorgegebenen Messtisch bekannter Dimensionen vorgenommen werden.

Der Gegenstand ist bei der Messung vorzugsweise fixiert. Sollte er dies nicht sein, wird vorzugsweise an dem Gegenstand eine Markeranordnung (z.B. ein Referenzstern) angebracht, der mit vermessen wird, um somit Bewegungen des zu vermessenden Gegenstandes zu berücksichtigen.

Wie bereits oben ausgeführt, wird bevorzugt die relative Lage mindestens von zwei Teilen des Gegenstands erfasst. Es kann natürlich auch die relative Lage zwischen beliebig mehr Teilen des Gegenstands erfasst werden, wie beispielsweise 3, 4, oder 5 Teile. Dass die relative Lage bestimmter Teile des Gegenstandes relative zu einem oder mehreren andern Teilen vermessen werden, schließt natürlich nicht aus, dass der gesamte Gegenstand vollständig vermessen wird, insbesondere seine äußere Gestalt nicht nur zum Teil sondern auch vollständig bestimmt wird.

Der Gegenstand wird vorzugsweise so platziert, dass sich das Instrument innerhalb der Körperstruktur befindet. Dabei bedeutet "innerhalb" insbesondere, dass ein Teil des Gegenstandes von der Körperstruktur umgeben ist. Insbesondere wird der Gegenstand relativ zur Körperstruktur ortsfest fixiert. Bei einer Ausführungsform kann aber auch eine Bewegung des Instruments, z.B. die Instrumentenspitze mit dem erfindungsgemäßen Verfahren bzw. der erfindungsgemäßen Vorrichtung nachverfolgt werden, indem ein sichtbarer Teil des Instruments wiederholt vermessen wird.

Falls hierin von der relativen Lage der Markereinrichtung (z.B. eine Markeranordnung, wie ein Referenzstern oder einzelne Marker) relativ zu einer Körperstruktur die Rede ist, ist dabei insbesondere die relative Lage zu vorbestimmten, insbesondere charakteristischen Teilen der Körperstruktur gemeint. Insbesondere ist die relative Lage der einzelnen Marker der Markereinrichtung relativ zu den vorbestimmten Teilen oder dem vorbestimmten Teil der Körperstruktur gemeint.

Im Folgenden wird eine Ausführungsform der Erfindung beschrieben. Dabei werden weitere Merkmale offenbart. Die offenbarten Merkmale können untereinander kombiniert werden. Soweit nicht anders bemerkt, bezeichnen gleiche Bezugszeichen gleiche Teile.

Die folgende Ausführungsform wird beispielhaft für den Fall der Verwendung bei einer Operation beschrieben. Der dabei erwähnte "Knochen" ist nur ein Beispiel für eine Körperstruktur. Der Begriff "Körperstruktur" umfasst auch künstliche Gliedmaßen, so dass die vorliegende Erfindung auch die Positionierung eines Instruments relativ zu einer künstlichen Gliedmaße, also nicht notwendigerweise während einer Operation erlaubt.
Figur 1a) zeigt einen Aufbau zum Erfassen von Lagemerkmalen eines Instruments;
Figur 1b) ist die Seitenansicht von Figur 1a);
Figur 2 zeigt eine Anordnung zum Bestimmen der relativen Lage eines Instruments relativ zu einem Knochen.
Figur 3a) zeigt eine Messanordnung zur Vermessung eines ersten Teils eines zweiteiligen Instruments;
Figur 3b) ist eine Seitenansicht der Figur 3a);
Figur 4a und 4b zeigen zwei Alternativen zur Vermessung einer Ebene.
Figur 5 zeigt schematisch eine Kette von relativen Lagen.

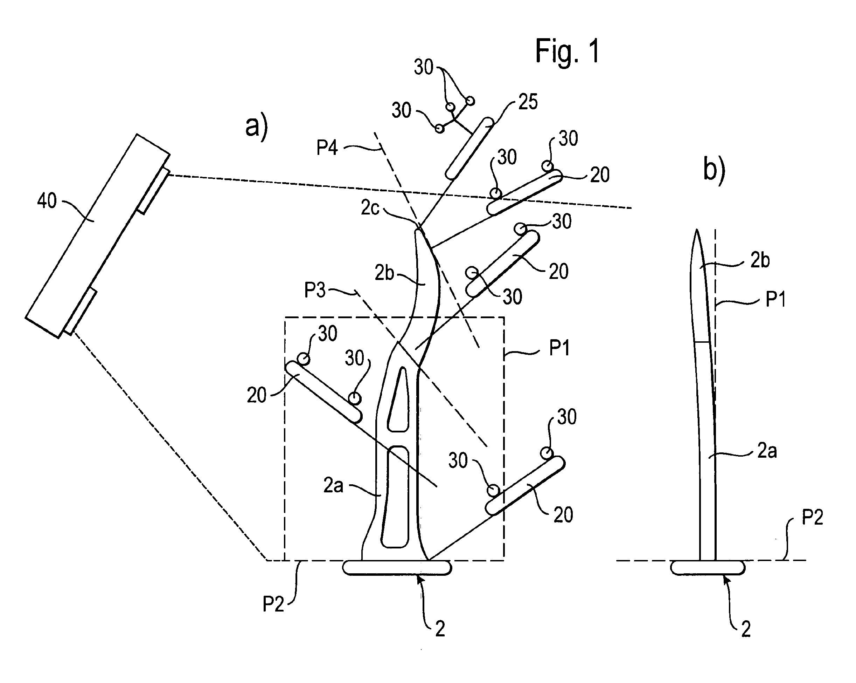

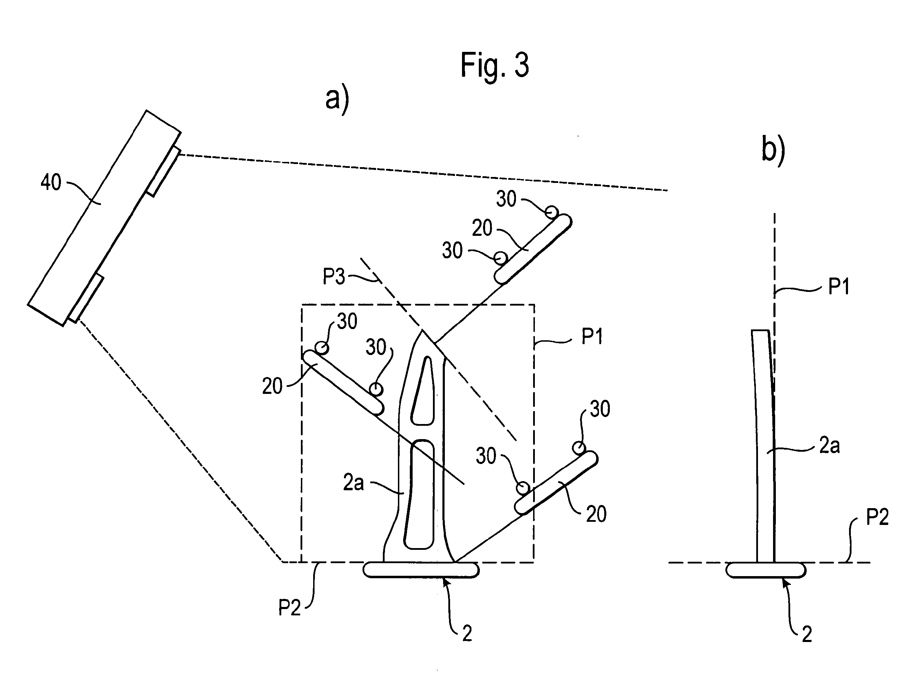

Vorzugsweise sind die Lagemerkmale eines Instruments, die die relative Lage von Teilen des Instruments zueinander beschreiben, bereits gespeichert. Die Figur 1 zeigt einen Messaufbau, der es erlaubt die (noch nicht gespeicherten) Lagemerkmale eines Instruments 2 zu vermessen, um sie anschließend zu speichern. Das in Figur 1 gezeigte Instrument 2 ist zweiteilig und besteht somit aus zwei Elementen, ein Element 2a und ein Element 2b. Die Elemente 2a und 2b können einstückig oder getrennt ausgebildet sein, insbesondere kann das Element 2b lösbar an dem Element 2a angebracht sein. Ist das Element 2b an dem Element 2a lösbar angebracht, so ist die relative Lage der Elemente 2a und 2b vorzugsweise fixiert, d.h. ortsfest. Später wird in Verbindung mit Figur 3 der Fall beschrieben, dass nur das erste Element 2a des Instruments vermessen wird. Natürlich kann das Instrument auch aus mehreren Elementen bestehen oder diese aufweisen.

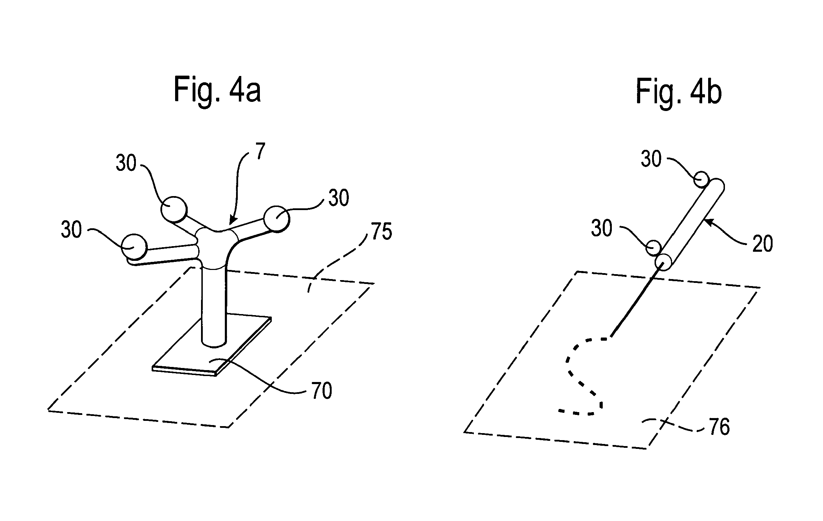

Wie weiter unten beschrieben wird, wird das Instrument 2 später teilweise, nämlich das Element 2b in einen Knochen eingeführt. Die Anordnung in Figur 1 erlaubt die Vermessung der Lagemerkmale des Instruments 2. Vorzugsweise werden charakteristische Ebenen erfasst, deren relative Lage als Lagemerkmale dienen und die z.B. im Verlaufe einer Operation einfach durch Anlegen eines Abtastmittels, insbesondere eines so genannten Pointers 20 bestimmt werden können. Bei dem Pointer handelt es sich insbesondere um ein Messinstrument mit insbesondere zwei daran angebrachten Markerkugeln 30. Die Vermessung einer Ebene mittels eines Pointers wird weiter unten in Verbindung mit Figur 4b erläutert. Eine Kamera 40 erfasst von den durch Kreisen dargestellten und an den Pointer 20 angebrachten Markern 30 ausgehendes Licht. Die Marker 30 können passive Marker sein, die Licht reflektieren oder aktive Marker, die Licht ausstrahlen. Natürlich können auch neben Licht (insbesondere Infrarotlicht) auch andere Wellen oder Strahlungen, wie zum Beispiel Ultraschall verwendet werden, die durch ein Detektionsgerät 40 erfasst werden können. Bei der gezeigten Ausführungsform handelt es sich bei dem Detektionsgerät 40 um eine Kamera. Als Abtastmittel kann neben einem Pointer beispielsweise auch ein Laser eingesetzt werden.

Die verschiedenen zu vermessenden Ebenen sind mit P1, P2, P3 und P4 bezeichnet. Die Figur 1b zeigt eine Seitenansicht der Figur 1a. Die Spitze des Pointers 20 wird in den verschiedenen Ebenen P1, P2, P3 und P4 bewegt, um die Ebenen und deren relative Lage zueinander im Raum zu erfassen bzw. abzutasten. Auch können Punkte des Instruments vermessen werden, insbesondere die Spitze des Instruments mittels der Spitze eines Pointers 25, der mit einem Referenzstern 30 versehen ist. Mit dem Pointer 25 kann die relative Lage der Spitze des Instruments relativ zu den anderen Ebenen bestimmt werden. Bei der Vermessung des Instruments 2 ist das Instrument vorzugsweise ortsfest. Alternativ kann natürlich an dem Instrument 2 beispielsweise ein Referenzstern fixiert werden, um die relative Lage der Ebenen oder Messpunkte auch bestimmen zu können, wenn das Instrument bewegt wird. Die vorliegende Erfindung hat jedoch insbesondere den Vorteil, dass sie auch bei Instrumenten Anwendung finden kann, bei denen Marker und/oder ein Referenzstern nicht oder nur schwer anbringbar sind. Eine Messeinrichtung zum Vermessen des Instruments weist vorzugsweise Abtastmittel, wie Pointer 20 und/oder Marker 30 und ein Detektionsgerät 40 auf.

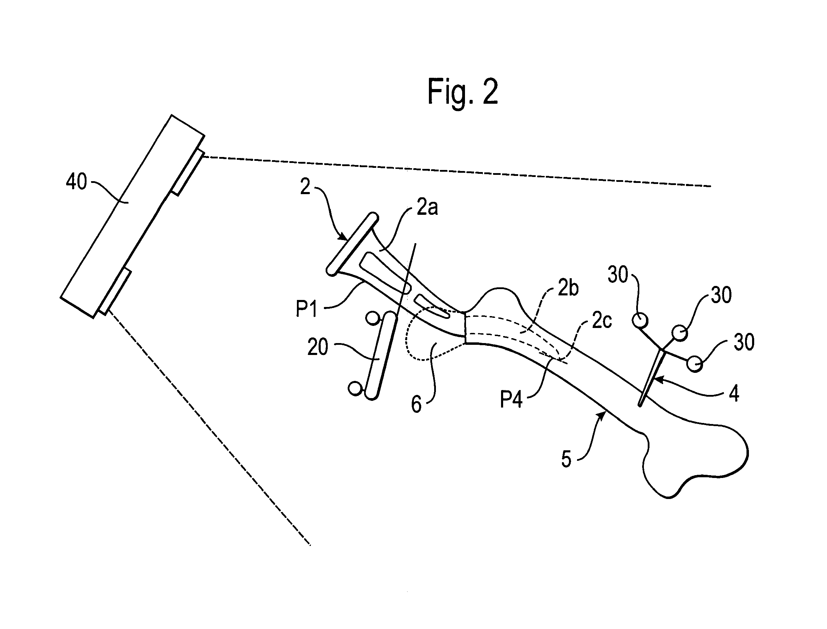

Figur 2 zeigt eine Anordnung zur Feststellung der Position des Instruments 2 relativ zu einem Knochen 5. Das Instrument 2 ist dabei mit seinem Element 2b innerhalb des Knochens 5 untergebracht. Das gezeigte Beispiel soll eine Hüftoperation darstellen, bei der der Oberschenkelkopf 6 reserziert wurde. Das zweite Element 2b des Instruments ist als Stecheisen ausgebildet und fest mit dem Knochen 5 verbunden, so dass die Lage des Instruments 2 relativ zum Knochen 5 fixiert ist. In dieser Situation interessiert den Operateur beispielsweise die relative Lage des Instruments 2 zum Knochen 5, insbesondere die relative Lage des verdeckten Elements 2b zum Knochen 5. Insbesondere ist für den Operateur die Lage der Spitze des Instruments oder die Orientierung der Ebene P4 relativ zum Knochen 5 von Interesse. Die Erfindung erlaubt in der in Figur 2 gezeigten Situation die Bestimmung beispielsweise der relativen Lage der Ebene P4 und/oder der Spitze 2c zu dem Knochen 5 durch Messung der relativen Lage von einem Teil (z.B. Ebene P1) oder mehreren Teilen des sichtbaren Elements 2a des Instruments mittels beispielsweise eines Pointers 20, der z.B. die Ebene P 1 abtastet, relativ zu der Markeranordnung 4. Die in Figur 1 gezeigte Kamera 40 wird auch bei der Situation der Figur 2 verwendet, um die mit Pointer 20 abgetastete Lage eines Teils (Ebene P1) des Elements 2a relativ zur Markeranordnung 4 zu erfassen. Die Kamera 40 stellt zusammen mit dem Pointer 20 ein Beispiel für ein zweites Erfassungsmittel dar.

Wie in Figur 2 gezeigt ist, ist ein Referenzstern 4 am Knochen 5 angebracht. Der Referenzstern 4 ist hinsichtlich seiner Lage relativ zum Knochen 5 fixiert. Beispielsweise ist der Referenzstern 4 in den Knochen 5 eingeschraubt. Die relative Lage zwischen Knochen 5 und Referenzstern 4 ist vorzugsweise bekannt und vorzugsweise gespeichert. Um die relative Lage zu bestimmen, kann der Knochen zusammen mit dem Referenzstern 4, der ein Beispiel für eine Markeranordnung darstellt, registriert bzw. vermessen werden. Dies bedeutet beispielsweise, dass der Knochen mittels eines CT (einer dreidimensionalen Röntgenaufnahme) zusammen mit den Markern 30 der Markeranordnung 4 vermessen wird, um so die relative Lage der Marker 30 zu Teilen des Knochens 5 zu bestimmen. Die Bestimmung mittels CT stellt nur ein Beispiel für ein erstes Erfassungsmittel dar. Alternativ kann dies beispielsweise auch erfolgen, indem die Oberfläche des Knochens und auch die der Markerkugeln 30 der Markeranordnung 4 mittels eines Pointers abgetastet wird, um die wesentliche Struktur des Knochens und die relative Lage zwischen dem Knochen und den mit dem Knochen verbundener Markerkugeln zu bestimmen. Auch ist natürlich eine optische Erfassung des Knochens 5 mittels einer Kamera oder mehreren Kameras zusammen mit der Markeranordnung 4 möglich.

Als Ausgangslage für eine Bestimmung der Lage des Instruments hat man nun Daten über die Lage von Teilen des Knochens 5 (z.B. Lage der Längsachse des Knochens 5, Lage bestimmter Oberflächenabschnitte bzw. -punkte oder Ebenen des Knochens 5) relativ zu der Markeranordnung 4.

Neben der Lage von Teilen des Knochens werden erfindungsgemäß, beispielsweise mit einem Pointer 20 (siehe Figur 2) die Lage von einem Teil oder mehreren Teilen des (sichtbaren) Elements 2a bestimmt. Diese Bestimmung erfolgt also vorzugsweise dann, wenn, wie zuvor ausgeführt wurde, das Instrument 2 fest mit dem Knochen 5 verbunden ist, und insbesondere das Element 2b des Instruments in den Knochen eingeführt ist. Die Lage kann relativ zu einem oder mehreren Teilen des Knochens 5 und/oder relativ zur Markeranordnung 4 und/oder in einem Bezugssystem bestimmt werden, das z.B. im Raum (z.B. OP-Saal) ruht. In dem genannten Bezugssystem können auch die Lage der Teile des Knochens 5 und/oder der Marker der Markeranordnung 4 bestimmt werden, um so die relative Lage des zugänglichen Elements relativ zur Markeranordnung 4 und damit relativ zum Knochen 5 zu bestimmen. Die Lage von Teilen des (nicht-sichtbaren) Elements 2b wird vorzugsweise nicht bestimmt, da diese nicht oder nur schwer mit beispielsweise Pointem zugänglich sind. Der Begriff "sichtbar" wird hierin in dem Sinne gebraucht, das das Element für eine Vermessung der Lage von Teilen des Elements zugänglich und/oder geeignet ist, insbesondere für eine Vermessung mittels von Hand zu bewegender Lagemesseinrichtungen oder Abtastmitteln (z.B. Pointern), die Teile der Elemente abtasten und/oder z.B. optisch erfassen.

Die Situation, in der das Instrument am Knochen fixiert ist und insbesondere ein Element des Instruments (Element 2b) in den Knochen eingeführt ist, wird im Folgenden als fixierte Situation bezeichnet. In dieser fixierten Situation wird die Lage eines Teils des Instruments bestimmt, insbesondere die Lage eines Teils des sichtbaren Elements 2a, insbesondere relativ zu der Markeranordnung 4. Ein zugängliches (auch als sichtbar bezeichnetes) Element stellt hierzu ein Beispiel für ein in der fixierten Situation vermessenes Element dar, wobei "vermessen" heißt, dass die Lage von einem oder mehreren Teilen des Elements in der fixierten Situation, insbesondere relativ zur Markeranordnung 4 gemessen wurde. Ein nicht-zugängliches (nicht-sichtbares) Element stellt hierin ein Beispiel für ein in der fixierten Situation nicht vermessenes Element dar, wobei "nicht vermessen" heißt, dass die Lage von einem oder mehreren Teilen des Elements in der fixierten Situation nicht gemessen wurde, insbesondere nicht gemessen werden konnte und insbesondere relativ zur Markeranordnung 4 nicht gemessen wurde.

In dieser fixierten Situation sind somit folgende Informationen, und insbesondere somit auch Daten gegeben: Die relative Lage von einem oder mehreren vorbestimmten Teilen des Knochens 5 relativ zur Markeranordnung 4 ist bekannt. Weiter sind Daten über die relative Lage von einem vorbestimmten Teil oder mehreren vorbestimmten Teilen eines in der fixierten Situation vermessenen Elements des Instruments relative zu einem vorbestimmten Teil oder mehreren vorbestimmten Teilen eines in der fixierten Situation nicht vermessenen Elements des Instruments bekannt. Außerdem ist die relative Lage zwischen dem einen vorbestimmten Teil oder den mehreren vorbestimmten Teilen des vermessenen Elements relativ zu der Markeranordnung 4 bekannt. Die somit bekannte Daten bzw. Informationen könne verwendet werden, um die relative Lage von dem einen vorbestimmten Teil oder den mehreren vorbestimmten Teilen des Knochens relativ zu dem vorbestimmten Teil oder den mehreren vorbestimmten Teilen des nicht zugänglichen Elements des Instruments zu bestimmen. Somit kann insbesondere die relative Lage des vorbestimmten Teils oder der vorbestimmten Teile des nicht-vermessenen Elements des Instruments relative zu dem vorbestimmten Teil des Knochens oder den vorbestimmten Teilen des Knochens bestimmt werden.

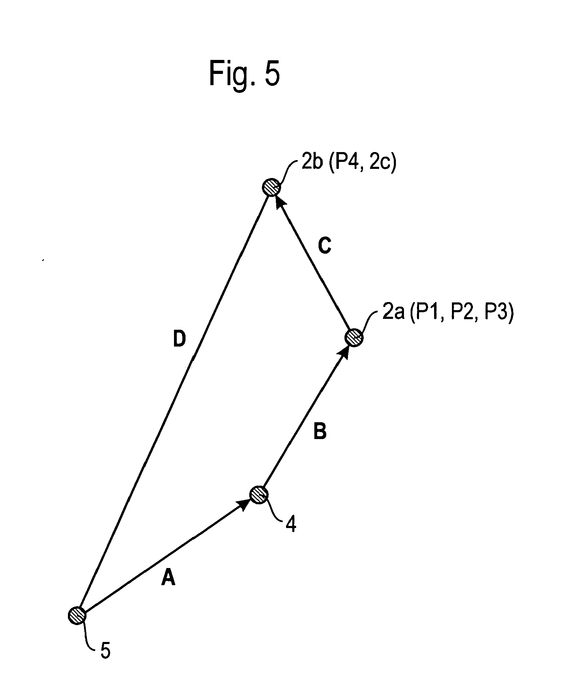

Die Bestimmung erfolgt vorzugsweise basierend auf einer Kette von relativen Lagen (siehe Figur 5), die wie folgt lautet: Die relative Lage A zwischen dem Knochen 5 und der Markeranordnung 4 ist bekannt. Die relative Lage B zwischen der Markeranordnung 4 und dem im fixierten Zustand vermessenen Element 2a des Instruments 2 ist bekannt. Die relative Lage C zwischen dem im fixierten Zustand vermessenen Element 2a und dem im fixierten Zustand nicht zugänglichen Element des Instruments 2 ist bekannt. Somit ergibt sich eine Kette aus relativen Lagen A, B und C, die es erlaubt die relative Lage D zwischen dem Knochen 5 und dem im fixierten Zustand nicht vermessenen Element 2b des Instruments 2 zu berechnen (siehe Fig. 5). Bei der vorgenannten Informationskette wird die Lage des Knochens vorzugsweise durch die vorbestimmten Teile des Knochens 5 festgelegt, wird die Lage der Markeranordnung 4 durch die Lage der Marker der Markeranordnung 4 vorzugsweise festgelegt und wird die Lage der vermessenen Elemente des Instruments durch die Lage der vorbestimmten Teile der Elemente vorzugsweise festgelegt. Um die relative Lage D zu berechnen, können beispielsweise die relativen Lagen A, B und C durch Vektoren beschrieben werden, wobei ein Vektor A vom Knochen 5 zur Markeranordnung 4 zeigt, eine Vektor B von der Markeranordnung 4 zum Element 2a zeigt und ein Vektor C vom Element 2a zum Element 2b zeigt. Ein die relative Lage D darstellender Vektor D ergibt sich dann durch Vektoraddition der Vektoren A, B und C. Somit kann ein Berechnungsprogramm durch Anwendung geometrischer Gesetzte die relative Lage D bestimmen.

Figur 3 zeigt eine alternative Ausführungsform, bei der die Lage von Teilen eines Elements 2a des Instruments 2 vermessen und bestimmt werden. Wie in der Figur 1, werden hierzu Pointer 20 mit Markerkugeln 30 verwendet, um, insbesondere charakteristische vorbestimmte Teile des Elements, wie beispielsweise Ebenen und insbesondere die relative Lage dieser vorbestimmten Teile zueinander zu messen. Das zweite Element des Instruments 2b ist in Figur 3 nicht gezeigt. Dieses ist lösbar an dem Instrument 2 anbringbar. Eine Mechanik zum Anbringen des Instrumentenelements 2b an dem Instrumentenelement 2a ist dabei vorzugsweise so gestaltet, dass die relative Lage des Instrumentenelements 2a zu dem Instrumentenelement 2b fixiert und vorbekannt ist. Insbesondere ist es nur möglich, das Element 2b in einer vorbekannten Lage relativ zum Element 2a an dem Element 2a zu befestigen. Weiter ist vorzugsweise mindestens eine relative Lage eines Teiles des Elements 2b relativ zu mindestens einem Teil des Elements 2a vorbekannt. Dadurch kann das Element 2a beispielsweise als wieder verwendbarer Handgriff verwendet werden, während das Element 2b ein austauschbares Element, insbesondere mit vorgegebener Form ist.

Figur 4a und 4b zeigen Beispiele zur Bestimmung der Lage einer Ebene, die ein Beispiel für ein Teil eines Gegenstandes oder einer Körperstruktur darstellt.

Figur 4a zeigt einen Referenzstern 7 an dessen unterem Ende eine ebene Platte 70 relativ zum Referenzstern 7 ortsfest angebracht ist. Mit der ebenen Platte 70 lässt sich die Lage eine gestrichelt eingezeichneten Ebene 75 beispielsweise eines Instruments oder eines Knochens erfassen, indem die Platte 70 auf die Ebene 75 flächig, insbesondere bündig aufgelegt wird.

Eine alternative Erfassung der Lage einer Ebene 76 ist in Figur 4b gezeigt. Die Spitze eines Pointers 20 tastet mehrer Punkte in der Ebene 76 ab. Mithilfe der Marker 30, deren Position durch eine Kamera erfassbar ist, lässt sich dann die Lage der Ebene 76 in einem Bezugssystem und/oder relativ zu einem anderen Teil (eines Gegenstandes oder einer Körperstruktur) bestimmen.

## Patentansprüche

1. Verfahren zum Bestimmen der relativen Lage eines Gegenstandes (2) relativ zu einer Körperstruktur, insbesondere zum Bestimmen der relativen Lage eines medizinischen Instruments relativ zu einer Körperstruktur (5), insbesondere auch wenn sich das Instrument teilweise innerhalb der Körperstruktur (5) befindet;
wobei das Verfahren die folgenden Schritte umfasst:
a) die relative Lage (C) eines ersten Teils (P1, P2, P3) des Gegenstandes (2) zu einem zweiten, vom ersten Teil verschiedenen Teil (P4, 2c) des Gegenstandes (2) wird bereitgestellt,
b) eine Markereinrichtung (4) wird an der Körperstruktur (5) angebracht;
c) die relative Lage (A) der Markereinrichtung (4) relativ zur Körperstruktur (5) wird erfasst;
d) der Gegenstandes (2) wird relativ zur Körperstruktur (5) platziert;
e) die relative Lage (B) des ersten Teils (P1, P2, P3) des Gegenstandes (2) relativ zur Markereinrichtung (4) wird erfasst;
f) die relative Lage (D) des zweiten Teils (P4, 2c) des Gegenstandes (2) relativ zur Lage der Körperstruktur (5) wird basierend auf der relativen Lage (C) des ersten Teils (P1, P2, P3) relativ zum zweiten Teil (P4, 2c), basierend auf der relativen Lage (A) der Markereinrichtung (4) relativ zur Körperstruktur (5) und basierend auf der relativen Lage (B) des ersten Teils (P1, P2, P3) relativ zur Markeranordnung (4) berechnet.

2. Verfahren nach Anspruch 1, bei welchem zur Bereitstellung der relativen Lage gemäß Schritt a) die Lage des ersten Teils (P1, P3, P3) relativ zum zweiten Teil (P4, 2c) gemessen wird.

3. Verfahren nach Anspruch 1 oder 2, bei welchem der Gegenstand (2) mindestens ein erstes Element (2a) und ein zweites Element (2b) aufweist, wobei das erste Teil (P1, P2, P3) ein Teil des ersten Elementes (2a) ist und das zweite Teil (P4, 2c) ein Teil des zweiten Elements (2b) ist.

4. Verfahren nach Anspruch 3, bei welchem das zweite Element (2b) lösbar am ersten Element (2a) so anbringbar ist, dass sich eine vorbekannte relative Lage zwischen dem ersten Teil (P1, P2, P3) und dem zweiten Teil (P4, 2c) ergibt.

5. System zum Bestimmen der relativen Lage eines Gegenstandes relativ zu einer Körperstruktur, insbesondere zum Bestimmen der relativen Lage eines medizinischen Instruments relativ zu einer Körperstruktur, insbesondere auch wenn sich das Instrument teilweise innerhalb der Körperstruktur befindet;
wobei das System folgendes umfasst:
a) Bereitstellungsmittel zum Bereitstellen der relative Lage (C) eines ersten Teils (P1, P2, P3) des Gegenstandes (2) zu einem zweiten, vom ersten Teil verschiedenen Teil (P4, 2c) des Gegenstandes (2);
b) eine Markereinrichtung (4);
c) erstes Erfassungsmittel zum Erfassen der relativen Lage (A) der Markereinrichtung (4) relativ zur Körperstruktur (5), wenn die Markereinrichtung relativ zur Körperstruktur (5) ortsfest ist;
d) zweites Erfassungsmittel (40, 20, 30, 70) zum Erfassen der relativen Lage (B) des ersten Teils (P1, P2, P3) des Gegenstandes (2) relativ zur Markereinrichtung (4);
e) Berechnungsmittel zum Berechnen der relativen Lage (D) des zweiten Teils (P4, 2c) des Gegenstandes (2) relativ zur Lage der Körperstruktur (5) basierend auf der relativen Lage (C) des ersten Teils (P1, P2, P3) relativ zum zweiten Teil (P4, 2c), basierend auf der relativen Lage (A) der Markereinrichtung (4) relativ zur Körperstruktur (5) und basierend auf der relativen Lage (B) des ersten Teils (P1, P2, P3) relativ zur Markeranordnung (4).

6. System nach Anspruch 5, bei welchem der Gegenstand (2) mindestens ein erstes Element (2a) und ein zweites Element (2b) aufweist, wobei das erste Teil (P1, P2, P3) ein Teil des ersten Elementes (2a) ist und das zweite Teil (P4, 2c) ein Teil des zweiten Elements (2b) ist.

7. System nach Anspruch 6, bei welchem das zweite Element (2b) lösbar am ersten Element (2a) so anbringbar ist, dass sich eine vorbekannte relative Lage zwischen dem ersten Teil (P1, P2, P3) und dem zweiten Teil (P4, 2c) ergibt.

8. System nach einem der Ansprüche 5 bis 7, bei welchem die Markereinrichtung (4) zum ortsfesten Anbringen an einer Körperstruktur ausgebildet ist.

9. System nach einem der Ansprüche 5 bis 8, bei welchem der Gegenstand (2) Vertiefungen und/oder Vorsprünge und/oder Markierungen zum Anlegen eines Abtastmittels (20, 25) an die Markereinrichtung (4) aufweist.

10. System nach einem der Ansprüche 5 bis 9, bei welchem das Bereitstellungsmittel einen Speicher umfasst, der die relative Lage (C)eines ersten Teils (P1, P2, P3) des Gegenstandes (2) relativ zu einem zweiten, vom ersten Teil verschiedenen Teil (P4, 2c) des Gegenstandes (2) speichert und/oder eine Messeinrichtung (40, 20, 25, 30) zum Messen dieser relativen Lage (C) umfasst.

11. Programm, das, wenn es auf einem Computer läuft oder in einen Computer geladen wird, den Computer veranlasst, die relative Lage (D) des zweiten Teils (P4, 2c) des Gegenstandes (2) relativ zur Lage der Körperstruktur (5) zu berechnen, wobei in die Berechnung folgende Daten einfließen:
die relative Lage (C) des ersten Teils (P1, P2, P3) relativ zum zweiten Teil (P4, 2c),
die relative Lage (A) der Markereinrichtung (4) relativ zur Körperstruktur (5) und
die relative Lage (B) des ersten Teils (P1, P2, P3) relativ zur Markeranordnung (4) und
wobei zur Berechnung geometrische Gesetze angewendet werden.
